(19) Europäisches Patentamt / European Patent Office / Office européen des brevets

(11) **EP 3 601 319 B1**

(12) **FASCICULE DE BREVET EUROPEEN**

(45) Date de publication et mention de la délivrance du brevet:
**23.12.2020 Bulletin 2020/52**

(51) Int Cl.:
***C07K 14/405*** *(2006.01)*  ***C07K 14/795*** *(2006.01)*
***C07K 1/14*** *(2006.01)*

(21) Numéro de dépôt: **18715223.6**

(22) Date de dépôt: **22.03.2018**

(86) Numéro de dépôt international:
**PCT/FR2018/050700**

(87) Numéro de publication internationale:
**WO 2018/172708 (27.09.2018 Gazette 2018/39)**

(54) **PROCÉDÉ DE PRÉPARATION D'UN EXTRAIT LIQUIDE DE PHYCOBILIPROTEINES, EN PARTICULIER DE PHYCOCYANINE, À PARTIR DE CYANOBACTERIES OU DE MICROALGUES ET EXTRAIT AINSI OBTENU**

VERFAHREN ZUR HERSTELLUNG EINES FLÜSSIGEN EXTRAKTES AUS PHYCOBILIPROTEINEN, INSBESONDERE PHYCOCYANIN, AUS CYANOBAKTERIEN ODER MIKROALGEN UND SO ERHALTENER EXTRAKT

METHOD FOR PREPARING A LIQUID EXTRACT OF PHYCOBILIPROTEINS, IN PARTICULAR PHYCOCYANIN, FROM CYANOBACTERIA OR MICROALGAE AND EXTRACT THUS OBTAINED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.03.2017 FR 1752452**

(43) Date de publication de la demande:
**05.02.2020 Bulletin 2020/06**

(73) Titulaire: **Algosource**
**44602 Saint-Nazaire Cedex (FR)**

(72) Inventeurs:
• **JUBEAU, Sébastien**
**44350 Guerande (FR)**
• **PRUVOST, Jérémy**
**44250 Saint Brevin Les Pins (FR)**
• **JAOUEN, Pascal**
**44510 Le Pouliguen (FR)**
• **JENCK, Jean**
**69110 Sainte Foy Les Lyon (FR)**
• **LEPINE, Olivier**
**44400 Reze (FR)**

(74) Mandataire: **Ipsilon**
**Europarc - Bat B7**
**3, rue Edouard Nignon**
**44300 Nantes (FR)**

(56) Documents cités:
**FR-A1- 2 453 199   FR-A1- 2 863 615**
**FR-A1- 2 929 957**

• **SINGH N K ET AL: "Optimization of medium components for increased production of C-phycocyanin from Phormidium ceylanicum and its purification by single step process", BIORESOURCE TECHNOLOGY, ELSEVIER BV, GB, vol. 100, no. 4, 1 février 2009 (2009-02-01), pages 1663-1669, XP025680547, ISSN: 0960-8524, DOI: 10.1016/J.BIORTECH.2008.09.021 [extrait le 2008-10-26]**
• **ROBABEH VALI AFTARI ET AL: "The Optimized Concentration and Purity of Spirulina platensis  C-Phycocyanin: A Comparative Study on Microwave-Assisted and Ultrasound-Assisted Extraction Methods : Extraction Modeling to Optimize the Phycocyanin", JOURNAL OF FOOD PROCESSING AND PRESERVATION, vol. 39, no. 6, 1 décembre 2015 (2015-12-01), pages 3080-3091, XP055420032, TRUMBULL, CT, US ISSN: 0145-8892, DOI: 10.1111/jfpp.12573**
• **SILVEIRA ET AL: "Optimization of phycocyanin extraction from Spirulina platensis using factorial design", BIORESOURCE TECHNOLOGY, ELSEVIER BV, GB, vol. 98, no. 8, 26 janvier 2007 (2007-01-26), pages 1629-1634, XP005738206, ISSN: 0960-8524, DOI: 10.1016/J.BIORTECH.2006.05.050**

**Description**

DOMAINE DE L'INVENTION

La présente invention concerne le domaine des procédés d'extraction de cyanobactéries ou de microalgues en vue d'obtenir des composés biologiquement actifs, plus particulièrement pour obtenir des extraits riches en phycocyanine.

ART ANTERIEUR

De nombreuses méthodes ont été développées ces dernières années pour extraire différents types de molécules de cyanobactéries, dont une des plus connue et utilisée est la spiruline.

En effet la spiruline est riche en acides aminés essentiels et en protéines (près de 50 % massique), en fer, et aussi en glucides et lipides (acide gamma-linoléique) et en caroténoïdes (bêta-carotène). Elle renferme en outre de nombreuses vitamines, notamment des vitamines B1, B2, B6, B9, B12, et E, ainsi que des oligo-éléments minéraux (calcium, magnésium, zinc, potassium...). L'intérêt d'extraire ces molécules est grandissant pour des utilisations dans les domaines alimentaires, cosmétiques et/ou thérapeutiques.

Parmi les protéines, les phycobiliprotéines sont des pigments qui captent l'énergie lumineuse. Le principal pigment de la spiruline, la phycocyanine, est d'un bleu intense. Les phycobiliprotéines comprennent également l'allophycocyanine et la phycoérythrine

Dans la suite du texte, par "phycocyanine" on entend "phycobiliprotéines, en particulier la phycocyanine".

Les méthodes d'extraction de la phycocyanine à partir de la spiruline sont nombreuses. Cependant la plupart de ces procédés utilisent comme matériau de départ de la spiruline séchée (par exemple le brevet CN 101560254, ou la publication de Robabeh et al, 2015, Journal of food processing and préservation, 39, pp 3080-3091, ou de Silveira et al, 2007, Bioresource Technology, 98, pp 1629-1634) et/ou éventuellement broyée (FR 2789389), ou bien, si l'extraction est réalisée à partir de spiruline fraîche, les procédés comportent une étape de séchage ou de lyophilisation. Or il s'avère qu'à l'état sec la spiruline, et donc la phycocyanine, sont altérées et leurs propriétés bioactives sont dégradées.

D'autres procédés d'extraction de phycobiliprotéines sont décrits dans FR 2 863 615 et Singh et al. Bioresource Technology 100(2009) 1663-1699.

BUT DE L'INVENTION

Un premier but de l'invention est donc de proposer un procédé d'extraction de la phycocyanine, à partir de cyanobactéries fraîches, telles que la spiruline fraîche ou à partir de micro-algues, et qui ne comporte aucune étape dans laquelle le produit se retrouve à l'état sec.

Certains procédés d'extraction mettent en œuvre des solvants organiques pour l'extraction de la phycocyanine, tels que le polyéthylène glycol ou la glycérine (WO 2016/030643). Ces composés se retrouvent alors, au moins à l'état de traces, dans le produit final. La phycocyanine étant destinée à un usage alimentaire et/ou médical, il est souhaitable de ne pas utiliser de telles méthodes faisant intervenir des solvants organiques. En variante, le procédé doit mettre en œuvre des étapes de purification très coûteuses (chromatographie par exemple).

Un autre but de la présente invention est donc de proposer un procédé de préparation d'un extrait de phycocyanine ne faisant pas intervenir de solvants organiques, ni d'étapes de purification coûteuses.

On connait du document FR 2453199 un procédé d'extraction de phycocyanine mettant en œuvre des cyanobactéries fraîches, mais utilisant des quantités importantes de sels de calcium CaCl2, de carbonates et de sulfate d'alumine et conduisant après lyophilisation, à l'obtention d'une poudre bleue, pouvant être conservée et stockée.

Un autre but de la présente invention est de proposer un procédé de préparation qui limite l'ajout de sels, et permette d'obtenir un extrait de phycocyanine sous forme liquide, extrait qui soit stable dans le temps, sans nécessiter une lyophilisation ou un séchage final.

Un autre but du procédé de l'invention est d'éviter toute étape qui provoquerait la précipitation de la phycocyanine afin de conserver cette dernière en solution dans l'ensemble du procédé d'extraction, l'objectif principal étant d'obtenir une phycocyanine en solution, non dénaturée, pour lui conserver ses propriétés biologiques et sa biodisponibilité.

RESUME DE L'INVENTION

A cet effet, la présente invention propose un procédé de préparation d'un extrait liquide de phycobiliprotéines, et en particulier de phycocyanine, en solution, à partir de cyanobactéries ou de micro-algues renfermant de la phycocyanine, comprenant les étapes successives suivantes :

- i) une étape de lyse cellulaire, par une méthode physique ou mécanique, d'une suspension aqueuse desdites cyanobactéries ou micro-algues fraîches,
- ii) une étape de macération du lysat obtenu à l'étape i) dans une solution de cations calcium en vue de libérer les molécules hydrosolubles dans l'espace extra cellulaire des cyanobactéries ou des micro-algues,
- iii) une ou plusieurs étapes de clarification et de concentration de la suspension pour isoler les molécules hydrosolubles, parmi lesquelles la phycocyanine,

l'ensemble de ce procédé étant réalisé à un pH compris entre 5 et 8,5, sans séchage et à une température inférieure ou égale à 25°C permettant à la phycocyanine de conserver sa structure spatiale, préservant ainsi ses propriétés biologiques et lui conférant une meilleure biodisponibilité.

L'extrait obtenu est donc un extrait aqueux, sans trace de solvant organique, puisqu'aucune étape dudit procédé de l'invention ne comporte l'introduction de telles molécules en vue de l'extraction de la phycocyanine.

Le procédé selon l'invention ne comporte ni étape de précipitation, ni d'étape de séchage, ce qui permet à la phycocyanine de rester en solution, de ne pas être dénaturée et de conserver sa structure spatiale, préservant ainsi ses propriétés biologiques et lui conférant une meilleure biodisponibilité, comme présenté plus loin dans les exemples.

Par "méthode physique ou mécanique", on entend le fait que cette étape i) de déstructuration ou lyse cellulaire ne comprend l'ajout d'aucun additif chimique ou biologique, à l'opposé notamment du procédé de FR 2929957 qui vise à obtenir des cyanobactéries fortement chargées en métal (spiruline-métal) et dont la première étape est une induction physiologique par application d'un stress et la suppression de l'oxygène au moyen de NaCl et de sulfite de sodium avant la mise en présence des cyanobactéries avec un métal divalent. Ceci conduit à une dénaturation partielle de la phycocyanine, ce qui va à l'encontre du procédé selon la présente invention.

De préférence, l'étape de déstructuration ou lyse cellulaire comprend une phase de congélation-décongélation.

La phase de congélation-décongélation permet en particulier la fragilisation des parois et membranes cellulaires, afin de faciliter ensuite l'extraction des métabolites intracellulaires. De manière avantageuse, la phase de congélation-décongélation comprend la congélation et la conservation des cyanobactéries ou micro-algues congelées à une température inférieure à -18°C, de préférence inférieure à -20°C, de préférence encore inférieure à -24°C pendant une durée allant de un jour à un an, de préférence de deux semaines à six mois ; cette congélation est suivie d'une étape de décongélation lente à une température supérieure à 0°C, et de préférence inférieure à 5°C, pendant plusieurs heures.

L'étape de macération du lysat obtenu à l'étape i) est préférentiellement effectuée sous agitation de ladite suspension thermostatée à une température comprise entre 10°C et 25°C pendant plusieurs heures: avantageusement pendant au moins 3 heures, de préférence pendant au moins 4 heures, de préférence encore pendant au moins 6 heures.

L'étape de clarification est avantageusement effectuée par centrifugation, puis récupération de la solution surnageante. Cette clarification a pour but d'abattre une grande partie de la fraction particulaire présente dans la suspension obtenue à l'étape ii). Ces particules sont essentiellement des débris cellulaires (parois, membranes) et des métabolites peu ou pas hydrosolubles (lipides, protéines hydrophobes, etc.). Il a été constaté que la présence de $CaCl_2$ favorise grandement la décantation des particules en augmentant leur vitesse de sédimentation lors de la centrifugation.

De manière avantageuse, la solution surnageante issue de l'étape de centrifugation est soumise à une étape de microfiltration, de préférence une microfiltration tangentielle, au moyen d'une membrane présentant un seuil de coupure compris entre 0,1 $\mu$m et 2 $\mu$m, de préférence entre 0,1 $\mu$m et 1,4 $\mu$m, de préférence encore compris entre 0,2 $\mu$m et 1 $\mu$m, puis récupération du filtrat.

Cette étape de microfiltration permet d'une part d'achever la clarification de l'extrait aqueux par l'élimination de toutes les particules non-décantées lors de la centrifugation, et d'autre part d'abattre la charge bactérienne du produit (filtration stérilisante).

La fraction hydrosoluble comprenant notamment des protéines dont la phycocyanine, les phycobiliprotéines, et des sucres vont traverser la membrane et se retrouver dans le filtrat (perméat).

Ce filtrat de l'étape de microfiltration est ensuite avantageusement soumis à une séparation par ultrafiltration, de préférence une ultrafiltration tangentielle, au moyen d'une membrane à seuil de coupure compris entre 1 et 50 kDa, de préférence compris entre 5 et 25 kDa, permettant de séparer la phycocyanine des petites molécules hydrosolubles et de recueillir une solution aqueuse enrichie en phycocyanine.

Ainsi cette opération constitue une étape de purification de la phycocyanine, car la phycocyanine est retenue sur la membrane qui laisse passer les autres molécules hydrosolubles plus petites (notamment peptides, petits sucres, sels). Elle permet également de réduire le volume de la solution clarifiée de phycocyanine, en la concentrant. Sous forme concentrée, les solutions de phycocyanine sont alors plus stables au cours d'un stockage frigorifique.

Ainsi, la solution enrichie en phycocyanine obtenue après l'étape d'ultrafiltration peut renfermer une concentration en phycocyanine supérieure ou égale à 0,5 g/L, de préférence supérieure ou égale à 1 g/L, de préférence encore supérieure ou égale à 2 g/L, de préférence encore supérieure ou égale à 10 g/L. La teneur en phycocyanine dans ladite solution de phycocyanine est avantageusement déterminée par mesure de la densité optique à une ou plusieurs longueurs d'onde comprise(s) entre 615 nm et 750 nm.

En ce qui concerne la solution aqueuse de cations divalents de l'étape ii), elle renferme avantageusement entre 10 mM et 100 mM, de préférence entre 10 mM et 60 mM, de préférence encore entre 15 mM et 55 mM, de cations divalents. L'ajout de la solution aqueuse de cations divalents permet une meilleure migration des molécules hydrosolubles dans l'espace extracellulaire. Les cations divalents sont des ions calcium, plus particulièrement sous la forme de chlorure de calcium.

Si nécessaire, le pH est ajusté à une valeur comprise entre 5 et 7,5, de préférence à une valeur comprise entre 5,5 et 7,0.

Le procédé de préparation d'un extrait liquide riche en phycocyanine selon la présente invention peut être réalisé à partir de cyanobactéries choisies parmi la spiruline *Arthrospira platensis, l'Aphanizomenon flos-aquae, ou le Phormidium molle,* ou toute autre cyanobactérie renfermant de la phycocyanine.

La présente invention concerne également un extrait liquide de cyanobactéries ou de micro-algues préparé au moyen du procédé décrit ci-dessus, comprenant une teneur en phycocyanine en solution, non dénaturée, supérieure ou égale à 1 g/L, de préférence supérieure ou égale à 2 g/L, de préférence encore supérieure ou égale à 10 g/L. Cet extrait aqueux est limpide et stable dans le temps, ne présentant pas de sédimentation pendant plusieurs mois de conservation (à température ambiante). Il peut être utilisé sous forme de boisson.

BREVE DESCRIPTION DES DESSINS

L'invention sera bien comprise à la lecture de la description suivante d'exemples de réalisation, en référence aux figures annexées dans lesquelles :

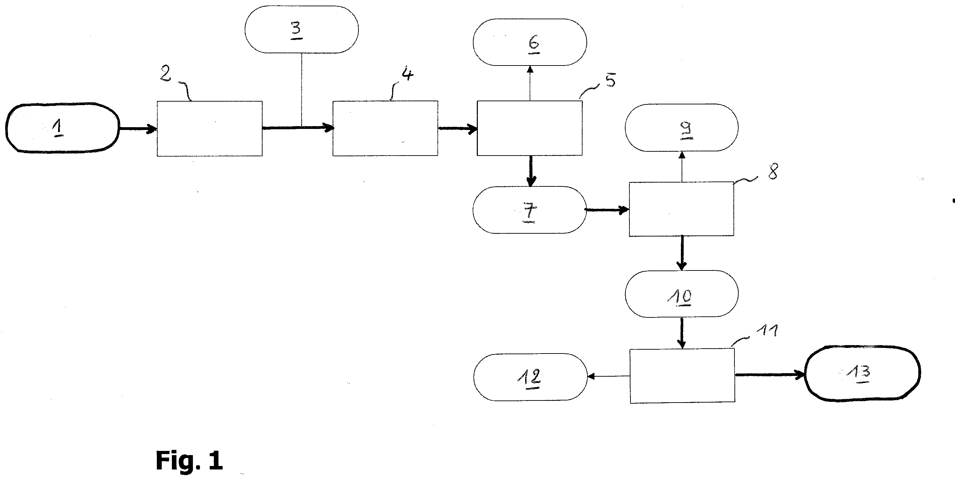

- La figure 1 est un diagramme schématique des principales étapes du procédé de préparation d'un extrait aqueux riche en phycocyanine selon la présente invention ;
- La figure 2 montre le rendement d'extraction de la phycocyanine en fonction du temps de macération pour une teneur en CaCl2 de 10 mM ;

- La figure 3 montre les résultats de mesure du statut anti-oxydant total (TAS) sur trois groupes de hamsters après 4 semaines et 11 semaines soumis à différents régimes dont l'un incluait l'extrait selon l'invention ;
- La figure 4 montre les résultats de mesure de la superoxyde dismutase (SOD) sur trois groupes de hamsters après 4 semaines et 11 semaines de régime ;
- La figure 5 montre les résultats de mesure de la glutathion peroxydase (GPx) sur trois groupes de hamsters après 11 semaines de régime ; et
- La figure 6 montre les résultats de mesure des niveaux plasmatiques de malondialdéhyde (MDA) sur trois groupes de hamsters après 11 semaines de régime.

EXEMPLES

**Exemple 1**

Dans cet exemple non limitatif illustrant l'invention, les cyanobactéries fraîches 1 de départ sont de la famille de la spiruline *Arthrospira platensis* cultivées dans des bioréacteurs en milieu aqueux saumâtre. Ces micro-algues sont collectées et égouttées sur un tamis de maille 50 $\mu$m. La teneur en matière sèche de cette biomasse est de 20 % massique.

La biomasse est tout à d'abord soumise à une étape de congélation 2 lente (inférieure à -1°C/min jusqu'à une température de -20°C) et conservée à cette température pendant 3 à 12 mois, avantageusement pour des raisons de productions saisonnières de la spiruline.

La conservation de la biomasse sous forme congelée présente le double avantage de s'assurer de la non-dénaturation de composés d'intérêt contenus dans les cellules mais également d'entraîner une déstructuration de la biomasse. Cette déstructuration résulte de différents phénomènes intervenants lors de la congélation à savoir la formation de glace intra et extracellulaire, une déshydratation osmotique et un réarrangement des cristaux de glace entrainant de fortes contraintes mécaniques sur les cellules. Ce dernier phénomène est assez lent et nécessite une durée de congélation significative afin de s'assurer que les principales barrières à l'extraction, que sont les différentes membranes et la paroi cellulaire, sont bien déstructurées. On s'assure ainsi, après décongélation, une bonne pénétration du solvant d'extraction et, par là même, de bons rendements d'extraction.

Après cette période de stockage à très basse température, la biomasse est décongelée jusqu'à une température supérieure à 0°C en la maintenant dans une enceinte à +3°C environ. Cette température basse, mais positive, autorise une lente décongélation favorisant la perméabilisation cellulaire.

Ensuite, l'extraction aqueuse a pour but de libérer la phycocyanine de l'enveloppe cellulaire afin de la retrouver en solution dans la phase aqueuse extracellulaire. A cet effet, la biomasse décongelée est tout d'abord mise en suspension dans une solution aqueuse (proportion massique solide/liquide : 15/85). Cette solution aqueuse 3 comprend de l'eau microfiltrée à 0,2 $\mu$m et du chlorure de calcium CaCl$_2$ à 10 mM. Le pH est ajusté si nécessaire à 7. La solution thermostatée à 20°C est soumise à une macération 4 sous agitation pendant 7 heures à l'obscurité.

La présence à la fois d'une grande quantité d'eau par rapport à la biomasse et du chlorure de calcium permet la migration de la majeure partie des molécules hydrosolubles dans l'espace extracellulaire. On retrouve alors dans cette fraction extraite plus de 95 %, voire 99 % de la phycocyanine présente dans la biomasse initiale (cf. exemple 2 ci-après).

Cette suspension est ensuite soumise à une étape de centrifugation 5 (15000 g, pendant 10 minutes à 20°C) qui permet d'écarter une grande partie de la fraction particulaire présente (débris cellulaires, et métabolites peu ou pas hydrosolubles). Il a été noté que la présence de chlorure de calcium favorise la décantation de ces particules. Le résidu d'algues 6 est écarté et n'est alors conservée que la solution surnageante 7 colorée en bleu.

Cette solution 7 est ensuite soumise à une nouvelle étape de clarification. Pour cela, une opération de microfiltration 8 tangentielle est réalisée. Le seuil de coupure est de 0,2 $\mu$m (il s'agit donc d'une filtration stérilisante). La fraction hydrosoluble (phycocyanine et sucres notamment) traverse cette membrane et se retrouve dans le perméat 10. Le rétentat 9 est évacué.

L'étape d'ultrafiltration 11 qui suit a pour but de concentrer le volume de cette solution de phycocyanine et d'éliminer les petites molécules contaminantes. Il s'agit d'une ultrafiltration tangentielle avec un seuil de coupure de 10 kDa. Ce seuil de coupure retient la phycocyanine tout en laissant passer dans le perméat 12 les autres molécules hydrosolubles plus petites (peptides, petits sucres, sels). Cette étape constitue donc également une étape de purification de la phycocyanine. On obtient ainsi une solution 13 limpide de phycocyanine concentrée et purifiée.

La teneur en phycocyanine (de plusieurs grammes par litre pouvant atteindre jusqu'à 50 g/L) est déterminée par mesure de la densité optique à 615 nm, 652 nm et 750 nanomètres (voir formule I de l'exemple 2). Cette solution de phycocyanine est stable pendant plusieurs mois sans ajout d'agent stabilisant ni de conservateur, et peut être conditionnée en ampoules stériles.

## Exemple 2 - Rendement en phycocyanine

De la pâte de spiruline congelée à environ 20% de matière sèche est placée dans de l'eau à température ambiante et à une concentration en CaCl2 de 10mM (1,1 g/L) selon le ratio 15/85 (m/m). Le mélange est ensuite placé à l'obscurité sous agitation.

Une première fraction de cette suspension obtenue est centrifugée (10 min, T$_{ambiante}$, 13000 x g) afin de suivre l'extraction de la phycocyanine (PC). L'extrait aqueux surnageant ainsi obtenu est ensuite analysé au spectrophotomètre par mesure de l'absorbance à différentes longueurs d'onde : respectivement 615 nm, 652 nm et 750 nm. La concentration en phycocyanine est calculée en utilisant l'équation (I) suivante (selon Bennett and Bogorad, J. Cell. Biol., 419-435, 1973) :

$$[PC]_{g/L} = \frac{(Abs_{615nm} - Abs_{750nm}) - 0{,}474(Abs_{652} - Abs_{750nm})}{5{,}34} \tag{I}$$

Afin de calculer les rendements d'extraction, la quantité totale de phycocyanine doit être déterminée. Une seconde fraction de la même suspension de spiruline a, pour ce faire, été traitée dans un destructeur cellulaire par hautes pressions dynamiques qui permet d'obtenir une lyse totale des cellules et une libération de toutes les molécules hydrosolubles contenues dans les cellules. Le broyat ainsi obtenu est ensuite clarifié puis analysé par spectrophotométrie comme les extraits aqueux précédents.

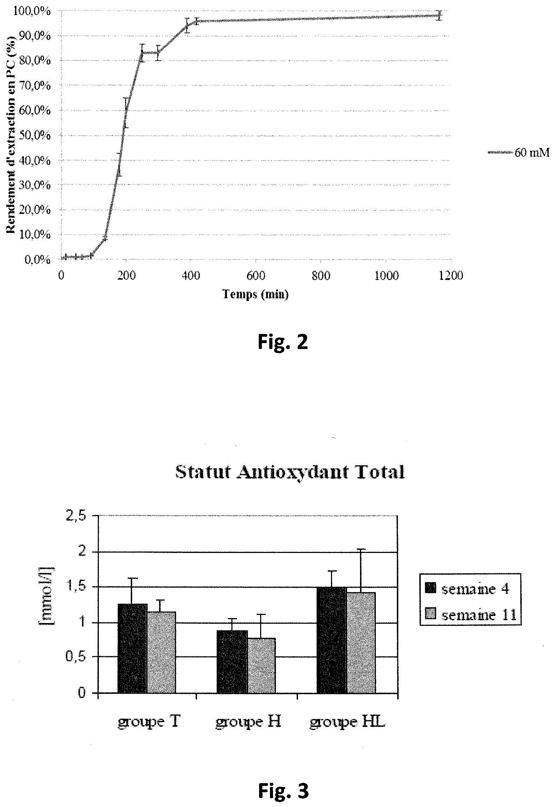

Les résultats montrant le rendement d'extraction de la phycocyanine en fonction du temps d'extraction sont présentés sur la figure 2 annexée.

Après 400 minutes d'extraction selon le protocole détaillé précédemment, on constate notamment que plus de 95% de la phycocyanine totale a été extraite suivant le procédé de la présente invention.

## Exemple 3 - Propriétés *in vitro*

Dans cet exemple, a été mesurée l'effet de la dégradation de la phycocyanine lors d'une étape de séchage, par mesure de l'activité SOD (Super Oxide Dismutase) au moyen du kit Bioxytech SOD-525 commercialisé par Cayman Chemical (USA).

Deux échantillons identiques de phycocyanine extraite selon le procédé décrit dans l'exemple 1 ci-dessus ont été comparés :

A - une ampoule de 5 ml de phycocyanine en solution renfermant 9,50 mg de phycocyanine. L'activité SOD équivalente mesurée est de 181 U/mL soit pour 10 mg de phycocyanine, une activité de 953 U SOD équivalente.

B - l'extrait de phycocyanine a été déshydraté à basse température, soit en dessous de 30°C, jusqu'à obtenir une poudre. Cette poudre renfermait 350 mg de phycocyanine par gramme. La mesure de l'activité SOD équivalente a indiqué une valeur de 633 U/g, soit ramené à 10 mg de phycocyanine, une valeur de 18 U SOD équivalente.

L'activité SOD équivalente de la phycocyanine n'ayant subi aucune étape de séchage est donc de 953/18 = 53 fois plus élevée que le même produit sec.

## Exemple 4 - Propriétés *in vivo*

Un extrait aqueux de spiruline a été préparé selon le procédé tel que décrit dans l'exemple 1. Cet extrait renferme 1g/L de phycocyanine.

L'activité antioxydante de cet extrait de spiruline a été démontrée par une étude réalisée durant 11 semaines sur trois groupes de 6 hamsters ayant suivi différents régimes :

- Groupe T : soumis à un régime standard
- Groupe H : soumis à un régime hyperlipidique
- Groupe HL : soumis à un régime hyperlipidique avec une supplémentation en spiruline liquide (phycocyanine à 1g/L : l'extrait de spiruline est fourni dans l'eau de boisson des hamsters à une dose de 1mL/jour).

Le régime hyperlipidique étant connu pour induire un stress oxydant, le bilan de ce stress est déterminé par différentes mesures effectuées en semaine n°4 et/ ou en semaine n°11 :

- Mesure du statut anti-oxydant total (TAS) :

Le statut anti-oxydant total mesuré avec le Kit TAS- NX 2332 (Randox Laboratories Ltd) reflète le potentiel d'activité du système anti-oxydant permettant de protéger les tissus contre les effets des radicaux libres. Les résultats présentés sur la figure 3 montrent que la supplémentation en extrait riche en phycocyanine conduit à un meilleur statut antioxydant, qui permet d'augmenter la capacité de la cellule à se défendre contre les dommages du stress oxydatif.

- Mesure de la superoxyde dismutase (SOD) :

Il s'agit de la première enzyme de la chaîne de réduction des radicaux libres. La SOD est l'enzyme nécessaire au maintien de la vie en présence d'oxygène, elle permet d'éliminer les espèces d'oxygène actives toxiques pour les cellules. La SOD est mesurée sur le sang au moyen du kit Ransod - SD 125 ((Randox Laboratories Ltd).

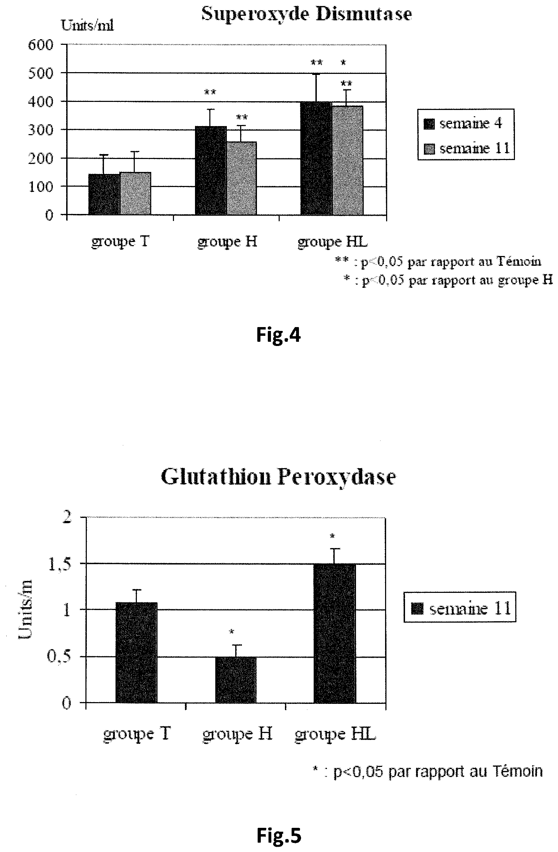

La quantité de SOD présente dans le sang est augmentée de 30 à 50 % chez les hamsters soumis au régime avec extrait de spiruline (voir figure 4). L'extrait de spiruline augmente la quantité de SOD disponible dans le sang en stimulant sa production.

- Mesure de la glutathion peroxydase (GPx) :

La glutathion peroxydase présente la propriété de réduire les radicaux libres oxydés en oxydant le glutathion réduit (GSH) en glutathion. Elle est mesurée avec le kit Ransel - RS 505 (Randox Laboratories Ltd).

A la semaine 11, on remarque une augmentation significative de l'activité GPx (voir figure 5) grâce à l'extrait de spiruline selon l'invention qui permet ainsi une lutte efficace contre les radicaux libres.

-Mesure de la peroxydation des lipides :

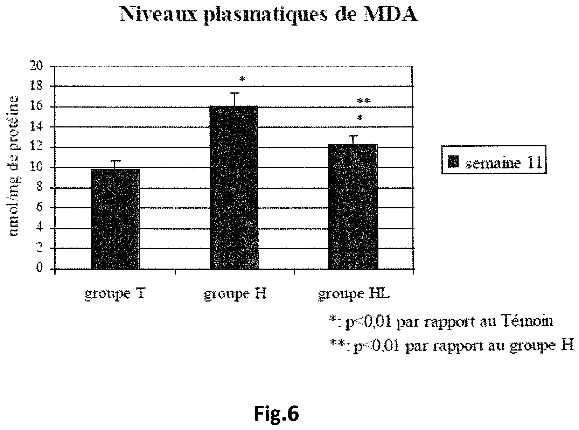

Le malondialdéhyde (MDA) est présent naturellement dans les tissus, c'est un des produits de l'oxydation des acides gras, un taux élevé est un marqueur de stress oxydant. Il est mesuré sur le plasma avec le kit MDA, réf. 1203.001 (Sobioda).
Les résultats présentés sur la figure 6 montrent que la supplémentation en extrait de spiruline permet une baisse du taux de MDA et donc une inhibition de la peroxydation des lipides permettant une atténuation du stress oxydant.

L'ensemble des résultats ci-dessus montrent donc que l'extrait de spiruline préparé selon le procédé de la présente invention possède une bonne biodisponibilité et une activité anti-oxydante démontrée *in vivo*.

**Revendications**

1. Procédé de préparation d'un extrait liquide de phycobiliprotéines, et en particulier de phycocyanine, en solution aqueuse, à partir de cyanobactéries ou de micro-algues renfermant de la phycocyanine, comprenant les étapes successives suivantes :

   - i) une étape de lyse cellulaire, par une méthode physique ou mécanique, d'une suspension aqueuse desdites cyanobactéries ou micro-algues fraîches,
   - ii) une étape de macération du lysat obtenu à l'étape i) dans une solution renfermant des ions calcium, en vue de libérer les molécules hydrosolubles dans l'espace extra cellulaire des cyanobactéries ou des micro-algues,
   - iii) une ou plusieurs étapes de clarification et de concentration de la suspension pour isoler les molécules hydrosolubles, parmi lesquelles la phycocyanine,

   l'ensemble de ce procédé étant réalisé à un pH compris entre 5 et 8,5, sans séchage et à une température inférieure ou égale à 25°C permettant à la phycocyanine en solution aqueuse de conserver sa structure spatiale, préservant ainsi ses propriétés biologiques et lui conférant une meilleure biodisponibilité.

2. Procédé selon la revendication 1, **caractérisé en ce que** l'étape de déstructuration ou lyse cellulaire comprend une phase de congélation-décongélation.

3. Procédé selon la revendication 2, **caractérisé en ce que** la phase de congélation-décongélation comprend la congélation et la conservation des cyanobactéries ou micro-algues congelées à une température inférieure à -18°C, de préférence inférieure à -20°C, de préférence encore inférieure à -24°C pendant une durée allant de un jour à un an, de préférence de deux semaines à six mois, suivie d'une étape de décongélation lente à une température supérieure à 0°C, et de préférence inférieure à 5°C, pendant plusieurs heures.

4. Procédé selon l'une quelconque des revendications précédentes, **caractérisé en ce que** l'étape de macération du lysat obtenu à l'étape i) est effectuée sous agitation de ladite suspension thermostatée à une température comprise entre 10°C et 25°C pendant plusieurs heures.

5. Procédé selon l'une quelconque des revendications précédentes, **caractérisé en ce que** l'étape de clarification est effectuée par centrifugation, puis récupération de la solution surnageante.

6. Procédé selon la revendication 5, **caractérisé en ce que** la solution surnageante issue de l'étape de centrifugation est soumise à une étape de microfiltration, de préférence une microfiltration tangentielle, au moyen d'une membrane présentant un seuil de coupure compris entre 0,1 $\mu$m et 2 $\mu$m, de préférence entre 0,1 $\mu$m et 1,4 $\mu$m, de préférence encore compris entre 0,2 $\mu$m et 1 $\mu$m, puis récupération du filtrat.

7. Procédé selon la revendication 6, **caractérisé en ce que** le filtrat de l'étape de microfiltration est soumis à une séparation par ultrafiltration, de préférence une ultrafiltration tangentielle, au moyen d'une membrane à seuil de coupure compris entre 1 et 50 kDa, de préférence compris entre 5 et 25 kDa, permettant de séparer la phycocyanine des petites molécules hydrosolubles et de recueillir une solution aqueuse enrichie en phycocyanine.

8. Procédé selon la revendication 7, **caractérisé en ce que** la solution enrichie en phycocyanine obtenue après l'étape d'ultrafiltration renferme une concentration en phycocyanine supérieure ou égale à 1 g/L, de préférence supérieure ou égale à 2 g/L, de préférence encore supérieure ou égale à 10 g/L, la teneur en phycocyanine dans ladite solution de phycocyanine étant déterminée par mesure de la densité optique à une ou plusieurs longueurs d'onde comprise(s) entre 615 nm et 750 nm.

9. Procédé selon l'une quelconque des revendications précédentes, **caractérisé en ce que** la solution aqueuse renfermant des ions calcium de l'étape ii) renferme entre 10 mM et 100 mM, de préférence entre 10 et 60 mM, de préférence encore entre 15 mM et 55 mM, d'ions calcium.

10. Procédé selon l'une quelconque des revendications précédentes, **caractérisé en ce que** le pH est ajusté à une valeur comprise entre 5 et 7,5, de préférence à une valeur comprise entre 5,5 et 7.

11. Procédé selon l'une quelconque des revendications précédentes, **caractérisé en ce que** les ions calcium sont ajoutés sous forme de CaCl2.

12. Procédé selon l'une quelconque des revendications précédentes, **caractérisé en ce que** les cyanobactéries sont choisies parmi la spiruline *Arthrospira platensis, l'Aphanizomenon flos-aquae, ou le Phormidium* molle.

13. Extrait liquide de cyanobactéries ou de micro-algues préparé au moyen du procédé selon l'une quelconque des revendications précédentes, comprenant une teneur en phycocyanine en solution aqueuse, non dénaturée, supérieure à 1 g/L, de de préférence supérieure ou égale à 2 g/L, de préférence encore supérieure ou égale à 10 g/L.

**Patentansprüche**

1. Verfahren zur Herstellung eines flüssigen Extrakts aus Phycobiliproteinen und insbesondere aus Phycocyanin in wässriger Lösung, aus Cyanobakterien oder aus Mikroalgen, die Phycocyanin enthalten, umfassend die folgenden aufeinanderfolgenden Schritte:

   - i) einen Zelllyseschritt anhand einer physikalischen oder mechanischen Methode einer wässrigen Suspension der frischen Cyanobakterien oder Mikroalgen,
   - ii) einen Mazerationsschritt des in Schritt i) erhaltenen Lysats in einer Lösung, die Calciumionen enthält, um die wasserlöslichen Moleküle im extrazellulären Raum der Cyanobakterien oder der Mikroalgen freizusetzen,
   - iii) einen oder mehrere Reinigungs- und Konzentrationsschritte der Suspension, um die wasserlöslichen Moleküle zu isolieren, darunter das Phycocyanin,

   wobei dieses gesamte Verfahren bei einem pH durchgeführt wird, der zwischen 5 und 8,5 liegt, ohne Trocknung und bei einer Temperatur von unter oder gleich 25 °C, was dem Phycocyanin in wässriger Lösung erlaubt, seine räumliche Struktur beizubehalten, wodurch seine biologischen Eigenschaften geschützt werden und was ihm eine bessere biologische Verfügbarkeit verleiht.

2. Verfahren nach Anspruch 1, **dadurch gekennzeichnet, dass** der Destrukturierungs- oder Zelllyseschritt eine Gefrier-Auftau-Phase umfasst.

3. Verfahren nach Anspruch 2, **dadurch gekennzeichnet, dass** die Gefrier-Auftau-Phase das Gefrieren und die Aufbewahrung der gefrorenen Cyanobakterien oder Mikroalgen bei einer Temperatur von unter -18 °C, vorzugsweise von unter -20 °C, noch vorzugsweiser von unter-24 °C während einer Dauer umfasst, die von einem Tag bis zu einem Jahr, vorzugsweise von zwei Wochen bis sechs Monate, reicht,
gefolgt von einem Schritt des langesamen Auftauens bei einer Temperatur von über 0 °C und vorzugsweise von unter 5 °C während mehrerer Stunden.

4. Verfahren nach einem der vorangehenden Ansprüche, **dadurch gekennzeichnet, dass** der Mazerationsschritt des in Schritt i) erhaltenen Lysats unter Rühren der auf einer Temperatur zwischen 10 °C und 25 °C thermostatierten Suspension während mehrerer Stunden durchgeführt wird.

5. Verfahren nach einem der vorangehenden Ansprüche, **dadurch gekennzeichnet, dass** der Reinigungsschritt durch Zentrifugieren, dann Rückgewinnen des Lösungsüberstands durchgeführt wird.

6. Verfahren nach Anspruch 5, **dadurch gekennzeichnet, dass** der aus dem Zentrifugierschritt hervorgegangene Lösungsüberstand einem Mikrofiltrationsschritt, vorzugsweise einer Tangentialmikrofiltration, mittels einer Membran unterzogen wird, die eine Trenngrenze aufweist, die zwischen 0,1 $\mu$m und 2 $\mu$m, vorzugsweise zwischen 0,1 $\mu$m und 1,4 $\mu$m, noch vorzugsweiser zwischen 0,2 $\mu$m und 1 $\mu$m, liegt, dann Zurückgewinnen des Filtrats.

7. Verfahren nach Anspruch 6, **dadurch gekennzeichnet, dass** das Filtrat des Mikrofiltrationsschritts einer Separierung durch Ultrafiltration, vorzugsweise einer Tangentialultrafiltration, mittels einer Membran mit einer Trenngrenze unterzogen wird, die zwischen 1 und 50 kDa, vorzugsweise zwischen 5 und 25 kDa, liegt, die es erlaubt, das Phycocyanin von den kleinen wasserlöslichen Molekülen zu separieren und eine mit Phycocyanin angereicherte wässrige Lösung zu gewinnen.

8. Verfahren nach Anspruch 7, **dadurch gekennzeichnet, dass** die nach dem Ultrafiltrationsschritt erhaltene, mit Phycocyanin angereicherte Lösung eine Phycocyaninkonzentration von über oder gleich 1 g/L, vorzugsweise von über oder gleich 2 g/L, noch vorzugsweiser von über oder gleich 10 g/L enthält, wobei der Phycocyaningehalt in der Phycocyaninlösung durch Messen der optischen Dichte bei einer oder mehreren Wellenlängen bestimmt wird, die zwischen 615 nm und 750 nm liegen.

9. Verfahren nach einem der vorangehenden Ansprüche, **dadurch gekennzeichnet, dass** die Calciumionen enthaltene wässrige Lösung von Schritt ii) zwischen 10 mM und 100 mM, vorzugsweise zwischen 10 und 60 mM, noch vorzugsweiser zwischen 15 mM und 55 mM Calciumionen enthält.

10. Verfahren nach einem der vorangehenden Ansprüche, **dadurch gekennzeichnet, dass** der pH auf einen Wert eingestellt wird, der zwischen 5 und 7,5 liegt, vorzugsweise auf einen Wert, der zwischen 5,5 und 7 liegt.

11. Verfahren nach einem der vorangehenden Ansprüche, **dadurch gekennzeichnet, dass** die Calciumionen in Form von CaCl2 hinzugefügt werden.

12. Verfahren nach einem der vorangehenden Ansprüche, **dadurch gekennzeichnet, dass** die Cyanobakterien aus der Spirulina *Arthrospira platensis, Aphanizomenon flos-aquae* oder dem *Phormidium molle* ausgewählt sind.

13. Flüssiger Extrakt aus Cyanobakterien oder Mikroalgen, hergestellt mittels des Verfahrens nach einem der vorangehenden Ansprüche, umfassend einen Gehalt an nicht denaturiertem Phycocyanin in wässriger Lösung von über 1 g/L, vorzugsweise von über oder gleich 2 g/L, noch vorzugsweiser von über oder gleich 10 g/L.

**Claims**

1. Method for preparing a liquid extract of phycobiliproteins, and of phycocyanin in particular, in aqueous solution, from cyanobacteria or microalgae containing phycocyanin, comprising the following successive steps:

   i) a cell lysis step, via a physical or mechanical method, of an aqueous suspension of said fresh cyanobacteria or microalgae;
   ii) a maceration step of the lysate obtained at step i) in a solution containing calcium ions, for the purpose of releasing the water-soluble molecules into the extracellular space of the cyanobacteria or microalgae;
   iii) one or more clarification or concentration steps of the suspension to isolate the water-soluble molecules which include phycocyanin,

   all of this method being conducted at a pH of between 5 and 8.5, without drying and at a temperature lower than or equal to 25 °C allowing the phycocyanin in aqueous solution to maintain its spatial structure, thereby preserving the biological properties thereof and imparting better bioavailability thereto.

2. The method according to claim 1, **characterized in that** the cell disruption or lysis step comprises a freeze-thaw phase.

3. The method according to claim 2, **characterized in that** the freeze-thaw phase comprises the freezing and preserving of the frozen cyanobacteria or microalgae at a temperature lower than -18 °C, preferably lower than -20°C, more preferably lower than -24 °C for a time ranging from one day to one year, preferably from two weeks to six months, followed by a slow thaw step at a temperature higher than 0 °C and preferably lower than 5 °C for several hours.

4. The method according to any of the preceding claims, **characterized in that** the maceration step of the lysate obtained at step i) is conducted under agitation of said suspension thermostat-controlled at a temperature of between 10 °C and 25 °C for several hours.

5. The method according to any of the preceding claims, **characterized in that** the clarification step is conducted by centrifugation followed by recovery of the supernatant solution.

6. The method according to claim 5, **characterized in that** the supernatant solution derived from the centrifugation step is subjected to a microfiltration step, preferably tangential flow microfiltration, by means of a membrane having a cut-off threshold of between 0.1 $\mu$m and 2 $\mu$m, preferably between 0.1 $\mu$m and 1.4 $\mu$m, more preferably between 0.2 $\mu$m and 1 $\mu$m, followed by recovery of the filtrate.

7. The method according to claim 6, **characterized in that** the filtrate of the microfiltration step is subjected to separation via ultrafiltration, preferably tangential flow ultrafiltration, by means of a membrane having a cut-off threshold of between 1 and 50 kDa, preferably between 5 and 25 kDa, allowing separation of the phycocyanin from the small water-soluble molecules and collection of an aqueous solution enriched with phycocyanin.

8. The method according to claim 7, **characterized in that** the solution enriched with phycocyanin obtained after the ultrafiltration step has a phycocyanin concentration higher than or equal to 1 g/L, preferably higher than or equal to 2 g/L, more preferably higher than or equal to 10 g/L, the phycocyanin content in said phycocyanin solution being determined by measuring the optical density at one or more wavelengths of between 615 nm and 750 nm.

9. The method according to any of the preceding claims, **characterized in that** the aqueous solution containing calcium ions at step ii) contains between 10 mM and 100 mM, preferably between 10 and 60 mM, more preferably between 15 mM and 55 mM of calcium ions.

10. The method according to any of the preceding claims, **characterized in that** the pH is adjusted to a value of between 5 and 7.5, preferably to a value of between 5.5 and 7.

11. The method according to any of the preceding claims, **characterized in that** the calcium ions are added in the form of CaCl2.

12. The method according to any of the preceding claims, **characterized in that** the cyanobacteria are selected from among the spirulina *Arthrospira platensis, Aphanizomenon flos-aquae* or *Phormidium molle.*

13. Liquid extract of cyanobacteria or microalgae prepared with the method according to any of the preceding claims, having a non-denatured phycocyanin content in aqueous solution higher than 1 g/L, preferably higher than or equal to 2 g/L, more preferably higher than or equal to 10 g/L.

**Fig. 1**

**Fig. 2**

**Fig. 3**

## Superoxyde Dismutase

** : p<0,05 par rapport au Témoin
* : p<0,05 par rapport au groupe H

**Fig.4**

## Glutathion Peroxydase

* : p<0,05 par rapport au Témoin

**Fig.5**

### Niveaux plasmatiques de MDA

*: $p < 0,01$ par rapport au Témoin
**: $p < 0,01$ par rapport au groupe H

**Fig.6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 101560254
- FR 2789389
- FR 2863615
- WO 2016030643 A
- FR 2453199
- FR 2929957

**Littérature non-brevet citée dans la description**

- **ROBABEH et al.** *Journal of food processing and préservation,* 2015, vol. 39, 3080-3091
- **DE SILVEIRA et al.** *Bioresource Technology,* 2007, vol. 98, 1629-1634
- **SINGH et al.** *Bioresource Technology,* 2009, vol. 100, 1663-1699
- **BENNETT ; BOGORAD.** *J. Cell. Biol.,* 1973, 419-435